(19) Europäisches Patentamt
European Patent Office
Office européen des brevets

(11) Veröffentlichungsnummer: **0 249 176 A2**

# (12) EUROPÄISCHE PATENTANMELDUNG

(21) Anmeldenummer: **87108234.3**

(22) Anmeldetag: **06.06.87**

(51) Int. Cl.⁴: **C07D 241/08**, A61K 31/495

(30) Priorität: **11.06.86 DE 3619562**

(43) Veröffentlichungstag der Anmeldung:
**16.12.87 Patentblatt 87/51**

(84) Benannte Vertragsstaaten:
**CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Eckhard, Roske**
**Hillensheimer Strasse 4**
**D-6700 Ludwigshafen(DE)**
Erfinder: **Gerhard, Fritz, Dr.**
**Limburgstrasse 23**
**D-6701 Dannstadt-Schauerheim(DE)**

## (54) Verfahren zur Herstellung von 2,3-Dioxo-4-oxicarbonylpiperazinderivaten, neue 2,3-Dioxo-4-oxicarbonylpiperazinderivate und deren Verwendung.

(57) Es wird vorgeschlagen, ein Verfahren zur Herstellung von 2,3-Dioxo-4-oxicarbonylpiperazinderivaten der allgemeinen Formel I

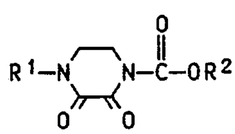

(I)

worin $R^1$ und $R^2$ unabhängig voneinander für aliphatische oder aromatische Kohlenwasserstoffreste stehen, unter Verwendung von in 4-Stellung unsubstituierten Piperazinderivaten und Chlorameisensäureestern, das dadurch gekennzeichnet ist, daß man in einer ersten Stufe 2,3-Dioxopiperazinderivat der allgemeinen Formel II

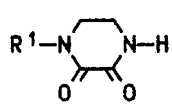

(II)

worin $R^1$ wie zuvor definiert ist, mit einem Chlorsilan der allgemeinen Formel III

$R_3SiCl$ (III)

worin R für einen Alkylrest steht, umsetzt, und das hierbei erhaltene silylierte Produkt in einer zweiten Stufe mit einem Chlorameisensäureester der allgemeinen Formel (IV)

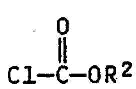

(IV)

worin $R^2$ wie zuvor definiert ist, umsetzt.

EP 0 249 176 A2

**Verfahren zur Herstellung von 2,3-Dioxo-4-oxicarbonylpiperazinderivaten, neue 2,3-Dioxo-4-oxicarbonylpiperazinderivate und deren Verwendung.**

Die Erfindung betrifft ein Verfahren zur Herstellung von 2,3-Dioxo-4-oxicarbonylpiperazinderivaten, neue 2,3-Dioxo-4-oxicarbonylpiperazinderivate und deren Verwendung bei der Herstellung halbsynthetischer Antibiotika, insbesondere halbsynthetischer Penicilline und Cephalosporine.

Bestimmte 2,3-Dioxopiperazinderivate sind Zwischenprodukte für die Synthese halbsynthetischer Antibiotika, welche in der Seitenkette 2,3-Dioxopiperazinreste aufweisen. Es handelt sich hierbei insbesondere um Penicillin-und Cephalosporin-Antibiotika. Ein Beispiel ist das Piperacillin, vergleiche Helwig, Moderne Arzneimittel, Nachtrags-und Ergänzungsband 1982, Seite 63, Wissenschaftliche Verlagsgesellschaft mbH, Stuttgart, 1982.

Nach dem Stand der Technik werden im allgemeinen zur Herstellung entsprechender Zwischenprodukte 1-Alkyl-2,3-dioxopiperazin-4-carbonylchloride eingesetzt. So beschreibt beispielsweise die DE-A-25 19 400 die Herstellung von 1-Ethyl-2,3-dioxo-4-piperazinocarbonylchlorid durch Umsetzung von 1-Ethyl-2,3-dioxo-piperazin mit Trimethylsilylchlorid in Gegenwart von Triethylamin und anschließende Umsetzung mit Phosgen bei 5 bis 10°C.

Nachteilig an den 1-Alkyl-2,3-dioxopiperazin-4-carbonylchloriden ist ihre starke Feuchtigkeitsempfindlichkeit. Die Verbindungen müssen unter Feuchtigkeitsausschluß gelagert werden und sind trotzdem wegen allmählicher Zersetzung nur sehr begrenzt haltbar. Die Reinigung der Säurechloride bereitet große Probleme, überdies erfordert der Umgang mit Phosgen einen erhöhten Sicherheitsaufwand.

In der DE-A-25 19 400 ist auch die Möglichkeit angedeutet, in 4-Stellung unsubstituierte Piperazine durch Reaktion mit dem Trichlormethylester der Chlorameisensäure in ein entsprechendes reaktives Derivat der 4-Carboxylverbindung zu überführen. Weitere Angaben, beispielsweise über Reaktionsbedingungen und Ausbeute, finden sich in der Druckschrift jedoch nicht, zumal der Schwerpunkt der bekannten Lehre auf der Herstellung der entsprechenden Säurehalogenide liegt.

Der Erfindung liegt die Aufgabe zugrunde, ein Verfahren zur Herstellung von 2,3-Dioxo-4-oxicarbonylpiperazinderivaten der allgemeinen Formel I zur Verfügung zu stellen:

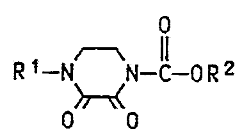

(I)

worin $R^1$ und $R^2$ unabhängig voneinander für aliphatische oder aromatische Kohlenwasserstoffe stehen, wobei in 4-Stellung unsubstituierte Piperazinderivate und Chlorameisensäureester verwendet werden, das die Herstellung der gewünschten Produkte in hohen Ausbeuten bei guter Reinheit erlaubt.

Diese Aufgabe wird gelöst bei einem Verfahren der genannten Art, das dadurch gekennzeichnet ist, daß man in einer ersten Stufe ein 2,3-Dioxopiperazinderivat der allgemeinen Formel II

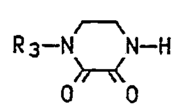

(II)

worin $R^1$ wie zuvor definiert ist, mit einem Chlorsilan der allgemeinen Formel III

$R_3SiCl$ (III)

worin R für einen Alkylrest steht, umsetzt, und das hierbei erhaltene silylierte Produkt in einer zweiten Stufe mit einem Chlorameisensäureester der allgemeinen Formel (IV)

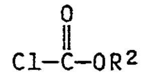

(IV)

worin $R^2$ wie zuvor definiert ist, umsetzt.

Das erfindungsgemäße Verfahren stellt die bis heute nicht zugänglichen 1-Alkyl-2,3-dioxo-4-alkyl-(ar)-oxycarbonyl-piperazine der allgemeinen Formeln (I) und I') zur Verfügung, welche überraschenderweise aus silyliertem 1-Alkyl-2,3-dioxopiperazin mit Chlorameisensäureestern hergestellt werden können.

Erfindungsgemäß lassen sich die 2,3-Dioxo-4-oxicarbonylpiperazinderivate der allgemeinen Formel (I) in hohen Ausbeuten bei guter Reinheit herstellen. Das Arbeiten mit Phosgen entfällt. Die Verbindungen besitzen eine ausgezeichnete Lagerstabilität, unbegrenzte Haltbarkeit, sind nicht empfindlich gegen Luftfeuchtigkeit und können auch bei Bedarf problemlos umkristallisiert werden.

Das erfindungsgemäße Verfahren eröffnet die Möglichkeit, auf direktem oder indirektem Weg Zwischenprodukte zu synthetisieren, welche die Einführung der jeweils gewünschten 2,3-Dioxo-4-oxicarbonylpiperazin-Seitenkette in halbsynthetische Antibiotika erlauben.

Nach einer bevorzugten Ausführungsform des erfindungsgemäßen Verfahrens führt man dieses bei Temperaturen zwischen 0 und 100°C, vorzugsweise zwischen 25 und 100°C, besonders bevorzugt zwischen 40 und 100°C durch. Wenn man in Gegenwart eines Lösungsmittels arbeitet, kann man, insbesondere bei der zweiten Stufe des Verfahrens, bei der Rückflußtemperatur des Systems arbeiten. In der zweiten Stufe arbeitet man besonders bevorzugt bei Temperaturen zwischen etwa 60 und 75°C.

Es ist zweckmäßig, das in der ersten Stufe erhaltene silylierte Produkt vor der weiteren Umsetzung mit dem Chlorameisensäureester zu isolieren und gegebenenfalls zu reinigen. So kann man das silylierte Produkt, welches im Reaktionsgemisch gelöst anfällt, nach gegebenenfalls durchgeführter Filtrierung durch Einengen, beispielsweise unter Vakuum, ausfällen, und auf übliche Weise, beispielsweise durch Filtrieren, isolieren. Das so erhaltene silylierte Produkt läßt sich zweckmäßig ohne weitere Reinigung zum gewünschten Zwischenprodukt umsetzen.

Das silylierte Produkt entspricht der nachstehenden Formel (IV)

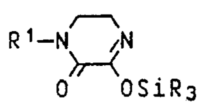

(IV)

worin die Reste R und $R^1$ die in den Ansprüchen angegebenen Bedeutungen besitzen.

Nach einer bevorzugten Ausführungsform des erfindungsgemäßen Verfahrens arbeitet man in Gegenwart eines inerten Lösungsmittels. Grundsätzlich ist jedes Lösungsmittel geeignet, das unter den Reaktionsbedingungen inert ist. Besonders bevorzugte Lösungsmittel sind 1,4-Dioxan, Tetrahydrofuran und Diethylether. Nach einer zweckmäßigen Ausgestaltung des erfindungsgemäßen Verfahrens verwendet man in der ersten Stufe als Lösungsmittel Dioxan und in der zweiten Stufe Tetrahydrofuran.

Besonders zweckmäßig führt man die Reaktion der ersten Stufe in Gegenwart einer Base, insbesondere in Gegenwart eines Trialkylamins durch. Als Trialkylamin kann man insbesondere ein ($C_1$-$C_6$)-Trialkylamin, insbesondere Triethylamin, verwenden. Es ist zweckmäßig, einen geringen molaren Überschuß an Trialkylamin, bezogen auf das eingesetzte Dioxopiperazin, einzusetzen.

Die Umsetzung der ersten Stufe erfolgt zweckmäßig unter Anwendung eines leichten Überschusses an Silylierungsmittel, beispielsweise einem 1,1-bis 1,9-fachen molaren Überschuß, bezogen auf das 2,3-Dioxopiperazin. In der zweiten Stufe ist es ebenfalls bevorzugt, mit einem geringen Überschuß an Chlorameisensäureester zu arbeiten, besonders zweckmäßig verwendet man einen 1,3-bis 2,0-fachen molaren Überschuß, bezogen auf das silylierte Produkt.

Die Reste $R^1$ und $R^2$ in den Verbindungen der allgemeinen Formeln (I) und (II) stehen unabhängig voneinander für aliphatische oder aromatische Kohlenwasserstoffreste. Es kann sich hierbei um Alkylreste, Cycloalkylreste, Alkylcycloalkylreste, Arylreste, Aralkylreste und dergleichen handeln. Die Art der Substituenten $R^1$ und $R^2$ ist beim erfindungsgemäßen Verfahren nicht kritisch, solange - insbesondere was den Rest $R^2$ anbetrifft - keine sterischen Hinderungen auftreten. Wenn die Reste $R^1$ und $R^2$ für Alkyl stehen, so können sie insbesondere $C_1$-bis $C_{18}$-, besonders bevorzugt $C_1$-bis $C_{12}$-Alkyl bedeuten. Die Alkylreste können geradkettig oder verzweigt sein. Wenn die Reste $R^1$ und $R^2$ für Cycloalkyl stehen, können sie insbesondere für $C_5$-bis $C_{12}$-Cycloalkylreste stehen. Wenn die Reste $R^1$ und $R^2$ für Arylreste stehen, können sie insbesondere substituiertes oder unsubstituiertes Phenyl oder Naphthyl bedeuten.

Besonders bevorzugt steht der Rest $R^1$ für Methyl oder Ethyl, der Rest $R^2$ für Methyl, Ethyl und Phenyl.

Der Rest R im Chlorsilan der allgemeinen Formel (III) hat die Bedeutung Alkyl, insbesondere $C_1$-bis $C_{18}$-Alkyl, besonders bevorzugt $C_1$-bis $C_6$-Alkyl.

Besonders bevorzugte Ausführungsformen der erfindungsgemäß erhältlichen 2,3-Dioxo-4-oxicarbonylpiperazinderivate sind diejenigen der allgemeinen Formel (I'):

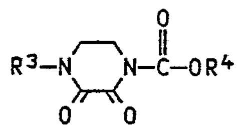

(I')

worin $R^3$ für einen $C_1$-$C_6$-Alkylrest steht und $R^4$ einen $C_1$-$C_6$-Alkylrest oder einen Phenylrest bedeutet.

Zu den $C_1$-$C_6$-Alkylresten gehören Methyl, Ethyl, n-Propyl, iso-Propyl, sec.-Butyl, tert.-Butyl, die verschiedenen isomeren Pentyl-und Hexylreste. Besonders bevorzugte Reste $R^3$ sind Methyl und Ethyl. Besonders bevorzugte Reste $R^4$ sind Methyl, Ethyl und Phenyl.

Als bevorzugtes Chlorsilan der allgemeinen Formel (III) wird das Trimethylchlorsilan verwendet.

Die Erfindung wird durch die nachstehenden Beispiele noch weiter erläutert.

## Beispiel 1

185 g (1,3 Mol) 1-Ethyl-2,3-dioxopiperazin werden in 3 l wasserfreiem 1,4-Dioxan gelöst, bei 25°C mit 182,4 g (1,68 Mol) Trimethylsilylchlorid versetzt und es werden bei schwach exothermer Reaktion bei 25 bis 28°C 155,3 g (1,54 Mol) Triethylamin zudosiert. Es wird noch ca. 16 h bei 25°C nachgerührt, dann das ausgefallene Triethylaminhydrochlorid unter Feuchtigkeitsanschluß abgesaugt und der Filterrückstand mit etwas Dioxan nachgewaschen. Nach Einengen des klaren Filtrates unter Wasserstrahlvakuum erhält man 267,5 g silylierte Trockenmasse.,

267 g (~1,25 Mol) der Silylverbindung werden in 3 l wasserfreiem Tetrahydrofuran (THF) gelöst und bei etwa 72°C bei schwachem Rückfluß innerhalb von 2,5 h mit 169,5 g (1,79 Mol) Chlorameisensäuremethylester tropfenweise versetzt. Es wird noch 0,5 h bei Rückflußtemperatur nachgerührt und durch anschließende Lösungsmittelabtrennung kristallisiert. Man erhält 243 g (97,2 %) Rohausbeute. Nach Umkristallisation aus THF liegt das reine 1-Ethyl-2,3-dioxo-4-(methoxicarbonyl)-piperazin vom Fp. = 126 bis 127,5°C vor, dessen Struktur durch Röntgenstrukturanalyse bestätigt wird.

Elementaranalyse

| | C | H | N | O |
|---|---|---|---|---|
| gef. % | 48,1 | 6,0 | 14,1 | 32,0 |
| ber. % | 48,0 | 6,0 | 14,1 | 32,0 |

H-NMR ($CDCl_3$)

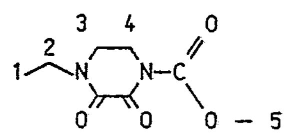

1. Triplett (3H) 1,2 ppm
2. Quadruplett (2H) 3,55 ppm
3. Triplett (2H) 3,62 ppm
4. Triplett (2H) 4,05 ppm
5. Singulett (3H) 3,92ppm

Beispiel 2

77 g (0,36 Mol) der Silylverbindung gemäß Beispiel 1, hergestellt wie in Beispiel 1 beschrieben, werden in 750 ml THF wasserfrei gelöst und bei 65 bis 68°C innerhalb von 2 h mit 61,2 g (0,56 Mol) Chlorameisensäureethylester unter Rühren versetzt. Man läßt dann bei Rückflußtemperatur nachreagieren. Danach konzentriert man unter Wasserstrahlvakuum auf und kristallisiert. Die Rohausbeute beträgt 68,2 g (88,3 %).

Das aus Diethylether/Ethanol umkristallisierte 1-Ethyl-2,3-dioxo-4-(ethoxicarbonyl)-piperazin schmilzt bei Fp. = 115 bis 116°C.

Elementaranalyse

| | C | H | N | O |
|---|---|---|---|---|
| gef. % | 50,5 | 6,6 | 13,0 | 30,0 |
| ber. % | 50,47 | 6,54 | 13,08 | 29,91 |

H-NMR ($CDCl_3$)

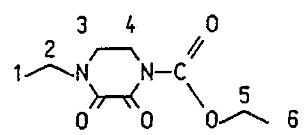

Triplett (3H) 1,2 ppm
1. Quadruplett (2H) 3,55 ppm
2. Triplett (2H) 3,62 ppm
3. Triplett (2H) 4,05 ppm
4. Quadruplett (2H) 4,36 ppm
5. Triplett (3H) 1,37 ppm.
6.

Beispiel 3

77 g (0,36 Mol) der Silylverbindung gemäß Beispiel 1, hergestellt wie in Beispiel 1 beschrieben, werden in 800 ml wasserfreiem THF gelöst. Zur Lösung dosiert man innerhalb von 2 h bei 66°C unter Rühren 90,4 g (0,58 Mol) Chlorameisensäurephenylester zu. Es wird noch 1 h nachreagieren gelassen, dann unter Wasserstrahlvakuum aufkonzentriert und kristallisiert. Die Rohausbeute beträgt 83,3 g (88,3 %).

Das aus Dioxan umkristallisierte 1-Ethyl-2,3-dioxo-4-(phenoxicarbonyl)-piperazin schmilzt bei Fp. = 165 bis 167°C.

Elementaranalyse

| | C | H | N | O |
|---|---|---|---|---|
| gef. % | 59,4 | 5,45 | 10,7 | 24,5 |
| ber. % | 59,54 | 5,34 | 10,69 | 24,43 |

H-NMR ($CDCl_3$)

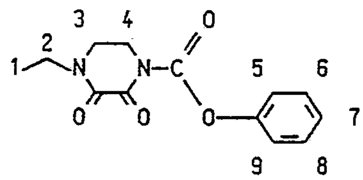

1. Triplett (3H) 1,2 ppm
2. Quadruplett (2H) 3,55 ppm
3. Triplett (2H) 3,62 ppm
4. Triplett (2H) 4,12 ppm
5., 9. Dublett (je 1H) 7,18 ppm
6., 8. Triplett (je 1H) 7,4 ppm
7. Triplett (1H) 7,26 ppm.

## Ansprüche

1. Verfahren zur Herstellung von 2,3-Dioxo-4-oxicarbonylpiperazinderivaten der allgemeinen Formel I

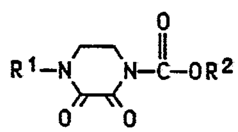

(I)

worin $R^1$ und $R^2$ unabhängig voneinander für aliphatische oder aromatische Kohlenwasserstoffreste stehen, unter Verwendung von in 4-Stellung unsubstituierten Piperazinderivaten und Chlorameisensäureestern, dadurch gekennzeichnet, daß man in einer ersten Stufe ein 2,3-Dioxopiperazinderivat der allgemeinen Formel II

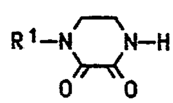

(II)

worin $R^1$ wie zuvor definiert ist, mit einem Chlorsilan der allgemeinen Formel III

$R_3SiCl$ (III)

worin R für einen Alkylrest steht, umsetzt, und das hierbei erhaltene silylierte Produkt in einer zweiten Stufe mit einem Chlorameisensäureester der allgemeinen Formel (IV)

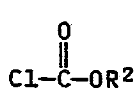

(IV)

worin $R^2$ wie zuvor definiert ist, umsetzt.

2. Verfahren nach Anspruch 1, dadurch gekennzeichnet , daß man bei Temperaturen zwischen 25 und 100°C arbeitet.

3. Verfahren nach einem der Ansprüche 1 oder 2, dadurch gekennzeichnet, daß man das in der ersten Stufe silylierte Produkt vor der weiteren Umsetzung isoliert.

4. Verfahren nach einem der Ansprüche 1 bis 3, dadurch gekennzeichnet, daß man in Gegenwart eines inerten Lösungsmittels arbeitet.

5. Verfahren nach Anspruch 4, dadurch gekennzeichnet, daß man als Lösungsmittel in der ersten Stufe 1,4-Dioxan und in der zweiten Stufe Tetrahydrofuran verwendet.

6. Verfahren nach einem der Ansprüche 1 bis 5, dadurch gekennzeichnet, daß man in der ersten Stufe in Gegenwart eines Trialkylamins arbeitet.

7. 2,3-Dioxo-4-oxicarbonyl-piperazinderivate der allgemeinen Formel I'

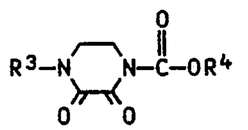

(I')

worin $R^3$ für einen $C_1$-$C_6$-Alkylrest steht und $R^4$ einen $C_1$-$C_6$-Alkylrest oder einen Phenylrest bedeutet.

8. 2,3-Dioxo-4-oxicarbonyl-piperazinderivate der allgemeinen Formel I' gemäß Anspruch 7, worin $R^3$ für Methyl oder Ethyl steht und $R^4$ Methyl, Ethyl oder Phenyl bedeutet.

9. Verwendung der Verbindungen der allgemeinen Formeln I und I' gemäß Ansprüchen 1 und 7 bei der Herstellung halbsynthetischer Antibiotika.